# EUROPÄISCHE PATENTSCHRIFT

(11) **EP 2 928 514 B1**
(45) Veröffentlichungstag und Bekanntmachung des Hinweises auf die Patenterteilung: **14.06.2017**
(21) Anmeldenummer: 13807891.0
(22) Anmeldetag: 05.12.2013
(51) Int. Cl.: A61M 1/00, A61L 9/00

(54) **ABSAUGVORRICHTUNG MIT SPÜLBARER DRAINAGELEITUNG**
ASPIRATING DEVICE HAVING A FLUSHABLE DRAINAGE LINE
DISPOSITIF D'ASPIRATION AVEC CONDUITE DE DRAINAGE BALAYABLE À L'AIR

(30) Priorität: 07.12.2012 CH 27322012
(43) Veröffentlichungstag der Anmeldung: 14.10.2015
(73) Patentinhaber: Medela Holding AG, 6340 Baar (CH)
(72) Erfinder: WALTI, Martin, CH-8038 Zürich (CH); EHLERT, Hilmar, CH-6052 Hergiswil (CH)
(74) Vertreter: Rutz, Andrea
(86) Internationale Anmeldenummer: PCT/CH2013/000214
(87) Internationale Veröffentlichungsnummer: WO 2014/085945

(56) Entgegenhaltungen:
- EP-A1- 2 170 022
- WO-A2-98/10774
- WO-A2-2007/087810
- US-A1- 2004 193 218
- US-A1- 2006 155 260
- US-A1- 2010 078 574

## Beschreibung

### TECHNISCHES GEBIET

Die vorliegende Erfindung betrifft eine Absaugvorrichtung zum Absaugen eines Fluids aus einem menschlichen oder tierischen Körper mittels Unterdruck. Derartige Absaugvorrichtungen finden z.B. Einsatz zur Pleuradrainage, zur Drainage des Mediastinums, zur Wunddrainage nach Verletzungen oder Operationen oder zur Absaugung von Körperfetten.

### STAND DER TECHNIK

Zum Absaugen von Körperflüssigkeiten und anderen Fluiden im medizinischen Bereich werden häufig Drainagepumpensysteme eingesetzt. Diese Systeme weisen üblicherweise eine Saugpumpe, einen oder mehrere Fluidsammelbehälter und einen Drainageschlauch zwischen Patient und Fluidsammelbehälter auf. Durch Erzeugen eines Unterdrucks im Fluidsammelbehälter wird Fluid vom Patienten über den Drainageschlauch in den Fluidsammelbehälter gesaugt und dort gesammelt.

Es ist bekannt, zusätzlich zum Drainageschlauch einen Hilfsschlauch zum Patienten zu führen. Dadurch kann der abzusaugenden Kavität des Patienten Luft zugeführt werden, und die Drainageleitung kann gespült werden. Ein Drainagepumpensystem mit Hilfsleitung ist z.B. aus der WO 2005/061025 bekannt. Auch die US 5,738,656 offenbart ein Drainagepumpensystem mit Hilfsleitung. Dort ist in der Hilfsleitung ein Bakterienfilter vorgesehen.

Die US 2006/0155260 offenbart ein Wunddrainagesystem, bei dem in einem geschlossenen Kreislauf die Wunde mit einer Spülflüssigkeit gespült wird, die Spülflüssigkeit zusammen mit dem Wundexsudat abgesaugt und gereinigt wird und der Wunde die gereinigte Spülflüssigkeit wieder zugeführt wird. Dabei kann die Spülflüssigkeit durch Einwirkung von Ultraviolett-, Gamma- oder Elektronenstrahlung entkeimt werden.

Die WO 2012/067918 schlägt vor, in einem Drainagepumpensystem die abgesaugte Flüssigkeit vor oder nach ihrem Eintritt in den Fluidsammelbehälter durch Einwirkung von Ultraviolettstrahlung zu entkeimen. Die derart behandelte Flüssigkeit wird anschliessend entsorgt.

In der US 2004/0193218 wird vorgeschlagen, optische Fasern in einer zu einer Wunde geführten Leitung vorzusehen, um ein Gas, das durch die Leitung der Wunde zugeführt wird, mittels UV-Licht zu sterilisieren.

Die EP 2 170 022 A1 offenbart eine Wunddrainageeinrichtung, bei welcher die Wunde mit einem nichtthermischen Plasma behandelt wird. Über eine Gasquelle kann der Wunde ein Gas zugeführt werden.

Das Dokument US 2010/0078574 A1 offenbart eine Entkeimungseinrichtung für Flüssigkeiten oder Gase, die als Durchflusszelle ausgestaltet ist, und bei der die Flüssigkeit oder das Gas durch die Einwirkung von UV-Strahlung sterilisiert wird.

Die WO 98/10774 A2 offenbart eine Vorrichtung zum Spülen von Körperhöhlen mit Ozon. Dazu ist eine Kammer vorgesehen, in welcher Luft ozoniert wird, um anschliessend der Körperhöhle zugeführt zu werden.

### DARSTELLUNG DER ERFINDUNG

Es ist eine Aufgabe der vorliegenden Erfindung, eine Absaugvorrichtung für Körperfluide anzugeben, mit der Luft durch eine Hilfsleitung zur Drainageleitung geführt werden kann, wobei die Gefahr verringert ist, dass der Patient durch diese Luft Infektionen erleidet.

Diese Aufgabe wird durch eine Absaugvorrichtung mit den Merkmalen des Anspruchs 1 gelöst. Vorteilhafte Ausgestaltungen sind in den abhängigen Ansprüchen angegeben.

Es wird eine Absaugvorrichtung zum Absaugen eines Fluids aus einem menschlichen oder tierischen Körper mittels Unterdruck vorgeschlagen. Die Absaugvorrichtung weist auf:
einen Drainageanschluss für eine Drainageleitung zum Abführen des Fluids aus dem Körper;
eine Vakuumquelle zur Erzeugung eines Unterdrucks, um das Fluid durch die Drainageleitung anzusaugen;
einen Fluidsammelbehälter zum Sammeln des durch die Drainageleitung angesaugten Fluids; und
einen Hilfsanschluss für eine Hilfsleitung.

Die Absaugvorrichtung weist ausserdem eine Entkeimungseinrichtung mit einer Entkeimungszelle auf. Die Entkeimungszelle hat einen Einlass, um ein noch unbehandeltes Gas, insbesondere Luft, zur Entkeimungseinrichtung zuzuführen. Die Entkeimungseinrichtung ist dazu ausgebildet, das der Entkeimungszelle zugeführte Gas derart zu behandeln, dass darin enthaltene Pathogene verändert und dadurch unschädlich gemacht werden, insbesondere, dass im Gas enthaltene Keime abgetötet werden. Die Entkeimungszelle hat ausserdem einen Auslass, um das behandelte Gas zum Hilfsanschluss abzuführen. Auslass und Einlass können physisch durch dieselbe Öffnung der Entkeimungszelle gebildet sein, sind aber vorzugsweise durch getrennte Öffnungen gebildet.

Die Entkeimungseinrichtung leistet weit mehr, als ein einfacher Bakterienfilter leisten kann: Sie filtert nicht einfach bestimmte Pathogene aus, sondern sie verändert Pathogene dauerhaft derart, dass sie unschädlich gemacht werden, d.h. dass ihre Pathogenität vermindert wird. Insbesondere tötet die Entkeimungseinrichtung Keime (replikationsfähige Pathogene) dauerhaft ab bzw. unterbindet deren Replikationsfähigkeit. Dadurch reduziert die Entkeimungseinrichtung nachhaltig die Belastung des Gases durch Pathogene und vermindert so die Einschleppung von Pathogenen in die Hilfsleitung. Ausserdem können auf diese Weise auch Pathogene entfernt werden, die durch einen Bakterienfilter hindurchdringen würden. So wird eine Einschleppung von Pathogenen in die nachfolgenden Leitungen wirksamer und nachhaltiger vermieden als durch einen Bakterienfilter. Dadurch wird die Gefahr verringert, dass der Patient durch das zugeführte Gas infiziert wird. Unter dem Begriff "Pathogene" werden dabei insbesondere Bakterien, Protozoen, Viren, Prionen und Pilzsporen verstanden.

Das zugeführte Gas ist vorzugsweise Umgebungsluft (Frischluft). Der Einlass ist dazu vorzugsweise mit dem Aussenraum verbunden, um die Umgebungsluft zur Entkeimungszelle zuzuführen. Dazu kann am Gehäuse der Saugvorrichtung oder der Entkeimungseinrichtung eine Luftzufuhröffnung vorhanden sein, die zum Aussenraum führt und die mit dem Einlass der Entkeimungszelle verbunden ist. Wenn es sich bei der Vakuumquelle um eine Pumpe handelt, wird der Entkeimungszelle vorzugsweise also nicht die von der Pumpe abgeführte Abluft aus dem Fluidsammelbehälter zugeführt, wie dies bei einem geschlossenen Kreislauf der Fall wäre, sondern Frischluft, deren Belastung durch Pathogene im Allgemeinen niedriger ist als die Abluft aus dem Fluidsammelbehälter. Es ist aber auch denkbar, Abluft zur Entkeimungszelle zuzuführen, wobei diese Abluft anschliessend wieder dem Körper zugeführt oder an den Aussenraum abgegeben wird.

Das der Entkeimungszelle zugeführte Gas wird einer äusseren Einwirkung physikalischer und/oder chemischer Natur ausgesetzt, um die darin enthaltenen Keime abzutöten. Vorzugsweise weist die Entkeimungseinrichtung eine Behandlungseinrichtung auf, um das zugeführte Gas mittels physikalischer Einwirkungen wie durch UV-Strahlen, Gammastrahlen, Elektronenstrahlen (z.B. Beta-Strahlung), durch Temperaturerhöhung und/oder durch Einwirkung von Wasserdampf zu behandeln. Dazu kann die Entkeimungseinrichtung als Behandlungseinrichtung mindestens eine UV-Lichtquelle, mindestens eine Wärmequelle und/oder mindestens einen Dampfgenerator umfassen. Es sind aber auch alternativ oder zusätzlich chemische Einwirkungen denkbar, insbesondere eine Ozonierung. Dazu kann die Entkeimungseinrichtung einen Ozongenerator umfassen.

In einer bevorzugten Ausgestaltung weist die Entkeimungseinrichtung am Einlass der Entkeimungszelle mindestens ein Einlassventil auf, durch welches das Gas zur Entkeimungszelle zuführbar ist, und am Auslass der Entkeimungszelle mindestens ein Auslassventil, durch welches das behandelte Gas aus der Entkeimungszelle abführbar ist. Die Absaugvorrichtung kann ausserdem eine Steuerungseinrichtung aufweisen, welche dazu ausgebildet ist, die folgenden Schritte durchzuführen:
(a) Schliessen des Auslassventils;
(b) Öffnen des Einlassventils, um das Gas zur Entkeimungszelle zuzuführen;
(c) Schliessen des Einlassventils;
(d) Behandeln des zugeführten Gases in der Entkeimungszelle derart, dass darin enthaltene Pathogene verändert und dadurch unschädlich gemacht werden; und
(e) Öffnen des Auslassventils, um das behandelte Gas aus der Entkeimungszelle abzuführen.

Dabei wird Schritt (b) vor, während oder nach Schritt (a) durchgeführt, d.h. das Einlassventil kann geöffnet werden, während das Auslassventil noch offen ist, es kann gleichzeitig mit dem Schliessen des Auslassventils geöffnet werden, oder es kann erst dann geöffnet werden, wenn das Auslassventil geschlossen ist. Die letzte Variante ist bevorzugt, d.h. die Ventile wirken bevorzugt wie eine Schleuse für das eintretende Gas. Schritt (c) wird nach Schritt (b) durchgeführt, und Schritt (d) (d.h. die Behandlung des Gases) wird zumindest in einem Teil des Zeitraums zwischen Schritt (c) und Schritt (e) durchgeführt, kann aber auch schon zuvor beginnen und/oder danach enden. Zwischen Schritt (c) und dem Ende von Schritt (d) liegen typischerweise wenige Sekunden bis einige Minuten, z.B. 1 Sekunde bis 10 Minuten. Zwischen dem Ende von Schritt (d) und Schritt (e) kann unter Umständen ein längerer Zeitraum liegen, in dem das behandelte Gas in der Entkeimungszelle verbleibt.

Um den Gasdurchsatz zu verbessern, kann die Entkeimungszelle ein veränderliches Volumen aufweisen. Dies ermöglicht es, das behandelte Gas möglichst vollständig aus der Entkeimungszelle auszustossen und eine möglichst grosse Menge an unbehandeltem Gas wieder aufzunehmen. Um das Volumen der Entkeimungszelle zu verändern, kann die Entkeimungszelle einen zylindrischen Wandbereich und einen darin verschiebbaren Kolben aufweisen. Es ist auch denkbar, dass die Entkeimungszelle einen faltenbalgartigen Wandbereich aufweist, um das Volumen der Entkeimungszelle zu verändern. Die Entkeimungszelle kann aber auch einen elastischen Wandbereich aufweisen und insbesondere ballonartig ausgebildet sein.

Das Volumen der Entkeimungszelle kann passiv veränderbar sein, indem sich das Volumen in Abhängigkeit vom Gasdruck in der Entkeimungszelle verändert. Um die Aufnahme des Gases in die Entkeimungszelle zu unterstützen, kann mindestens ein Federelement vorgesehen sein, das derart ausgebildet und angeordnet ist, dass es das Volumen zu maximieren trachtet. Dadurch kann die Entkeimungszelle so betrieben werden, dass sich das Volumen bei Anlegen eines genügend starken Unterdrucks gegenüber dem Umgebungsdruck unterhalb einer bestimmten Druckschwelle aufgrund der Druckdifferenz zum Aussenraum gegen die Federkraft selbsttätig verkleinert und bei einem Druckanstieg aufgrund der Federkraft wieder vergrössert.

Das Volumen der Entkeimungszelle kann aber auch aktiv veränderbar sein. Dadurch wird es möglich, gezielt unbehandeltes Gas aufzunehmen, unabhängig vom restlichen Betriebszustand der Absaugvorrichtung, und/oder behandeltes Gas gezielt und z.B. mit einem vorgebbaren Druck oder einer vorgebbaren Rate auszustossen. Zu diesem Zweck kann die Entkeimungseinrichtung einen Antrieb aufweisen, um das Volumen der Entkeimungszelle aktiv zu verändern. Dabei kann es sich z.B. um einen elektromotorischen oder pneumatischen Antrieb handeln.
In anderen Ausführungsformen ist es aber auch möglich, dass die Entkeimungszelle ein konstantes Volumen aufweist.
Die Entkeimungszelle kann so ausgebildet und angeordnet sein, dass durch sie ein kontinuierlicher, insbesondere im Wesentlichen konstanter, Volumendurchfluss stattfindet, wobei das die Entkeimungszelle durchströmende Gas kontinuierlich behandelt wird. Die Entkeimungszelle kann insofern als Durchflusszelle ausgebildet sein.
Als Vakuumquelle der Absaugvorrichtung dient vorzugsweise eine elektrisch betriebene Pumpe (z.B. eine Membranpumpe). Stattdessen kann aber z.B. zur Erzeugung des Unterdrucks auch ein Vakuumregulator zum Anschluss an ein Spitalvakuumsystem (z.B. über einen Wandanschluss) oder ein Venturi-System vorgesehen sein, wie dies aus dem Stand der Technik hinlänglich bekannt ist.
Die Absaugvorrichtung kann mit einer Drainageleitung, die mit dem Drainageanschluss verbunden ist, und einer Hilfsleitung, die mit dem Hilfsanschluss verbunden ist, komplettiert werden. Ein vorrichtungsfernes (d.h. im Betrieb körpernahes) Ende der Hilfsleitung steht dann vorzugsweise mit einem vorrichtungsfernen Ende der Drainageleitung in fluidkommunizierender Verbindung. Dazu kann eine direkte Verbindung zwischen den vorrichtungsfernen Enden der beiden Leitungen vorgesehen sein, oder die Verbindung kann z.B. über einen Hohlraum des Patienten erfolgen, indem die beiden Enden nahe beieinander im selben Hohlraum enden. Die entkeimte Luft aus der Entkeimungszelle kann dann insbesondere dazu dienen, die Hilfsleitung und dadurch auch die Drainageleitung zu spülen und gegebenenfalls Feststoffansammlungen, die sonst zu Verstopfungen führen könnten, in Richtung des Fluidsammelbehälters zu befördern.
Die Entkeimungszelle oder sogar die gesamte Entkeimungseinrichtung ist in die Pumpeneinheit der Absaugvorrichtung integriert. Dazu weist die Absaugvorrichtung ein Pumpaggregatsgehäuse auf, in dem die Pumpe angeordnet ist, und die Entkeimungszelle bzw. die gesamte Entkeimungseinrichtung ist dann ebenfalls im Pumpaggregatsgehäuse angeordnet und kann insbesondere in diesem vollständig aufgenommen sein. Die Entkeimungszelle bzw. die gesamte Entkeimungseinrichtung kann aber auch z.B. äusserlich am Pumpaggregatsgehäuse befestigbar sein. Die Entkeimungseinrichtung kann eine eigenständige, in sich geschlossene Einheit bilden. Die Entkeimungszelle oder die gesamte Entkeimungseinrichtung kann nachrüstbar und/oder austauschbar sein.

Die vorliegende Offenbarung bezieht sich auch auf eine Entkeimungseinrichtung als solche. Es wird also eine Entkeimungseinrichtung angegeben, die für eine Absaugvorrichtung zum Absaugen eines Fluids aus einem menschlichen oder tierischen Körper mittels Unterdruck geeignet ist. Die Entkeimungseinrichtung weist eine Entkeimungszelle auf, die mit einem Einlass versehen ist, um ein noch unbehandeltes Gas zur Entkeimungszelle zuzuführen. Die Entkeimungseinrichtung ist dazu ausgebildet, das zugeführte Gas derart zu behandeln, dass darin enthaltene Pathogene verändert und dadurch unschädlich gemacht werden. Die Entkeimungszelle weist ausserdem einen Auslass auf, um die behandelte Luft aus der Entkeimungszelle abzuführen.

Die obigen Überlegungen zur Absaugvorrichtung gelten sinngemäss auch für die Entkeimungseinrichtung alleine. Insbesondere kann das zugeführte Gas Umgebungsluft sein, und der Einlass kann dementsprechend mit einem Aussenraum verbunden sein, um Umgebungsluft zur Entkeimungseinrichtung zuzuführen. Es ist aber auch denkbar, Abluft aus der Absaugvorrichtung zur Entkeimungszelle zuzuführen, wobei diese Abluft anschliessend wieder dem Körper zugeführt oder an den Aussenraum abgegeben wird. Die Entkeimungseinrichtung kann eine Behandlungseinrichtung aufweisen, um das zugeführte Gas in der oben angegeben Weise zu behandeln, insbesondere mittels UV-Strahlen, durch Temperaturerhöhung und/oder durch Einwirkung von Wasserdampf. Sie kann ein Einlassventil aufweisen, durch welches das Gas zur Entkeimungszelle zuführbar ist, und ein Auslassventil, durch welches das behandelte Gas aus der Entkeimungszelle abführbar ist. Die Entkeimungszelle kann, wie oben näher beschrieben, ein veränderliches Volumen aufweisen. Dieses kann in der oben beschriebenen Weise passiv oder aktiv veränderbar sein.

Die vorliegende Offenbarung bezieht sich ausserdem auf ein Verfahren zum Zuführen von entkeimtem Gas zu einer Hilfsleitung einer Absaugvorrichtung, wobei die Absaugvorrichtung eine Drainageleitung aufweist, um ein Fluid aus einem menschlichen oder tierischen Körper mittels Unterdruck abzusaugen, und wobei ein vorrichtungsfernes Ende der Hilfsleitung mit einem vorrichtungsfernen Ende der Drainageleitung in fluidkommunizierender Verbindung steht. Das Verfahren umfasst:
Zuführen eines Gases zu einer Entkeimungszelle;
Behandeln des zugeführten Gases in der Entkeimungszelle derart, dass darin enthaltene Pathogene verändert und dadurch unschädlich gemacht werden; und
Abführen des behandelten Gases aus der Entkeimungszelle zur Hilfsleitung.

Das Gas ist vorzugsweise Umgebungsluft, d.h. es wird vorzugsweise Umgebungsluft aus dem Aussenraum zur Entkeimungszelle zugeführt. Es ist aber auch denkbar, Abluft aus der Absaugvorrichtung zur Entkeimungszelle zuzuführen, wobei diese Abluft anschliessend wieder dem Körper zugeführt oder an den Aussenraum abgegeben wird. Wie oben näher ausgeführt, kann das zugeführte Gas z.B. mittels UV-Strahlen, durch Temperaturerhöhung und/oder durch Einwirkung von Wasserdampf behandelt werden.

Wenn das Gas zur Entkeimungszelle durch ein Einlassventil zugeführt wird, und wenn das behandelte Gas aus der Entkeimungszelle durch ein Auslassventil abgeführt wird, kann das Verfahren insbesondere die folgenden Schritte umfassen:
(a) Schliessen des Auslassventils;
(b) Öffnen des Einlassventils, um das Gas zur Entkeimungszelle zuzuführen;
(c) Schliessen des Einlassventils;
(d) Behandeln des zugeführten Gases in der Entkeimungszelle derart, dass darin enthaltene Pathogene verändert und dadurch unschädlich gemacht werden; und
(e) Öffnen des Auslassventils, um das behandelte Gas aus der Entkeimungszelle abzuführen.

Bezüglich der Abfolge dieser Schritte gelten dieselben Überlegungen, die oben im Zusammenhang mit der Absaugvorrichtung ausgeführt wurden.

In dem Verfahren kann vorgesehen sein, dass das Volumen der Entkeimungszelle veränderlich ist, während das Gas zur Entkeimungszelle zugeführt und/oder das behandelte Gas aus der Entkeimungszelle abgeführt wird. Dies kann passiv durch Erzeugung von entsprechenden Druckdifferenzen oder aktiv durch einen Antrieb geschehen, wie dies oben näher ausgeführt wurde.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine schematische Darstellung eines ersten Ausführungsbeispiels einer Absaugvorrichtung mit Entkeimungszelle während der Anwendung für die Pleuradrainage;
- Fig. 2: eine schematische Darstellung der Entkeimungszelle der Absaugvorrichtung der Fig. 1 (Teil (a): Ausgangstellung; Teil (b): Entkeimungsstellung);
- Fig. 3: eine schematische Darstellung einer weiteren Ausführungsform einer Entkeimungszelle (Teil (a): Ausgangstellung; Teil (b): Entkeimungsstellung);
- Fig. 4: ein Diagramm zur Veranschaulichung der Zustandsabfolge der Ventile am Einlass und Auslass der Entkeimungszelle;
- Fig. 5: eine schematische Darstellung einer Drainage des Mediastinums;
- Fig. 6: eine schematische Darstellung einer Wunddrainage; und
- Fig. 7: eine schematische Darstellung eines weiteren Ausführungsbeispiels einer Absaugvorrichtung mit Entkeimungszelle.

### BESCHREIBUNG BEVORZUGTER AUSFÜHRUNGSFORMEN

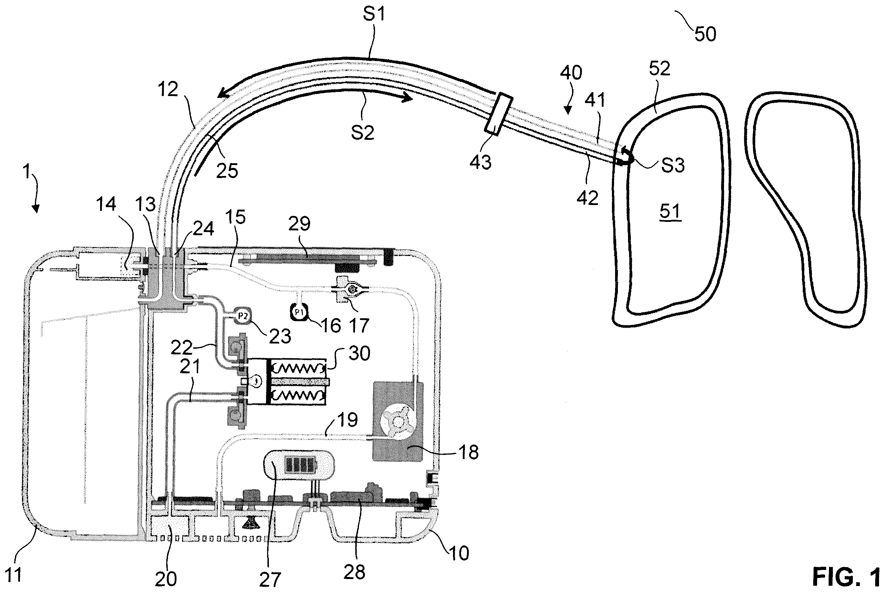

In der Fig. 1 ist eine erste Ausführungsform einer erfindungsgemässen Absaugvorrichtung illustriert. Die Absaugvorrichtung ist als kompakte, tragbare, batteriebetriebene, digitale Thoraxdrainagevorrichtung konzipiert. Sie umfasst ein Pumpaggregatsgehäuse 10, mit dem ein Fluidsammelbehälter 11 lösbar verbunden ist. Ein Drainageschlauch 12 mündet über einen Anschlussadapter, an dem ein Drainageanschluss 13 für den Drainageschlauch 12 ausgebildet ist, in den Fluidsammelbehälter 11. Über einen Filter 14 und eine Vakuumleitung 15 wird im Fluidsammelbehälter 11 mittels einer Saugpumpe 18 ein Unterdruck erzeugt. Ein Einwegventil 17 verhindert, dass Luft von der Vakuumpumpe zurück in den Fluidsammelbehälter 11 gelangt. Mittels eines Druckaufnehmers (Manometers) 16 kann der Druck P1 in der Vakuumleitung 15 festgestellt werden. Die von der Saugpumpe 18 angesaugte Luft wird über eine Abluftleitung 19 an den Aussenraum abgegeben. Bei der Saugpumpe 18 handelt es sich im vorliegenden Beispiel um eine motorbetriebene elektrische Vakuumpumpe, insbesondere um eine Membranpumpe bekannter Art.

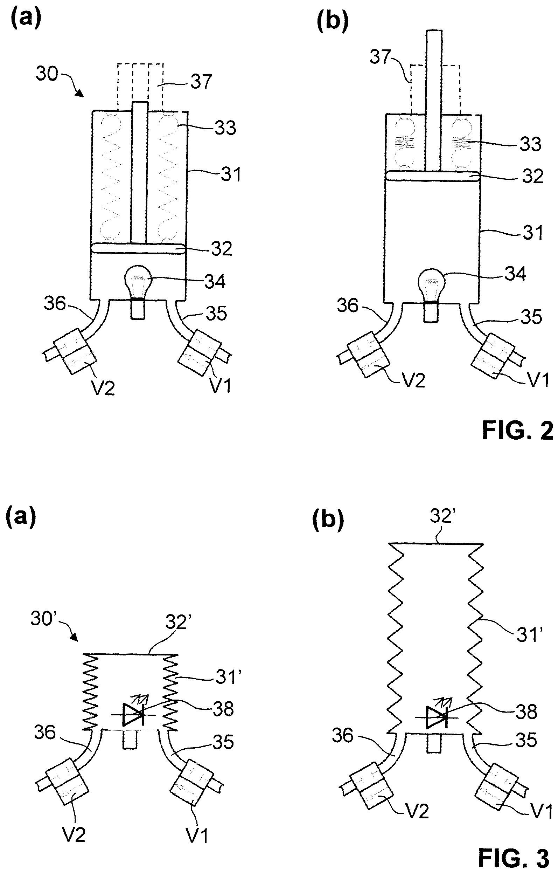

Im Pumpaggregatsgehäuse 10 ist eine Entkeimungszelle 30 aufgenommen, die nachfolgend noch näher im Zusammenhang mit der Fig. 2 beschrieben wird. Über einen Einlassfilter 20, der in einer Öffnung der Gehäusewand des Pumpaggregatsgehäuses 10 vorgesehen ist, und eine Einlassleitung 21 wird Frischluft aus dem Aussenraum zur Entkeimungszelle 30 zugeführt. Die in der Entkeimungszelle behandelte Luft wird über eine Auslassleitung 22 zu einem Hilfsanschluss 24 für einen Hilfsschlauch 25 geleitet. Der Druck P2 in der Auslassleitung 22 kann durch einen optionalen Druckaufnehmer 23 festgestellt werden.

Im Pumpaggregatsgehäuse 10 sind ausserdem weitere Komponenten angeordnet, die zum Betrieb der Absaugvorrichtung dienen. Insbesondere sind eine wiederaufladbare Batterie 27, eine digitale Steuerungseinrichtung 28 und ein Display 29 vorhanden. Die Steuerungseinrichtung 28 empfängt unter anderem die Messwerte von den Druckaufnehmern 16 und 23, steuert den Betrieb der Saugpumpe 18 und gibt Informationen zum Betriebszustand an das Display 29 aus.

Mit den vorrichtungsfernen Enden des Drainageschlauchs 12 und des Hilfsschlauchs 25 ist über einen Steckverbinder 43 ein doppellumiger Katheter 40 verbunden. Der Katheter 40 weist ein Drainagelumen 41 auf, das mit dem Drainageschlauch 12 verbunden ist, sowie ein Hilfslumen 42, das mit dem Hilfsschlauch 25 verbunden ist. Der Drainageschlauch 12 und das Drainagelumen 41 des Katheters 40 bilden gemeinsam eine Drainageleitung, während der Hilfsschlauch 25 und das Hilfslumen 42 gemeinsam eine Hilfsleitung bilden. Die Drainageleitung 12, 41 und die Hilfsleitung 25, 42 stehen an ihrem vorrichtungsfernen (körpernahen) Ende in fluidkommunizierender Verbindung, wie dies in der Fig. 1 durch den Doppelpfeil S3 angedeutet ist. Während die Verbindung im vorliegenden vereinfachten Beispiel ausserhalb des Katheters 40 erfolgt, kann auch eine direkte Verbindung zwischen den beiden Lumina 41, 42 innerhalb des Katheters 40 vorgesehen sein.

Im vorliegenden Beispiel dient die Absaugvorrichtung zur Thoraxdrainage (Pleuradrainage). Dazu mündet der Katheter 40 in den Pleuraraum 52, der einen Lungenflügel 51 eines Patienten 50 umgibt. Die Absaugvorrichtung dient dazu, einen Unterdruck im Pleuraraum 52 zu erzeugen, um den Pleuraspalt zu schliessen, und um Wundsekret aus dem Pleuraraum 52 abzusaugen. Im Normalbetrieb wird dazu die Saugpumpe 18 derart betrieben, dass sie im Fluidsammelbehälter 11 einen Unterdruck erzeugt. Dadurch werden Luft und Wundsekret über die Drainageleitung 12, 41 in den Fluidsammelbehälter 11 abgesaugt (Pfeil S1). Im Fluidsammelbehälter 11 werden flüssige und feste Bestandteile von der angesaugten Luft abgetrennt, und nur die angesaugte Luft gelangt über den Filter 14 und die Vakuumleitung 15 zur Saugpumpe 18 und wird über die Auslassleitung 19 nach aussen abgeführt.

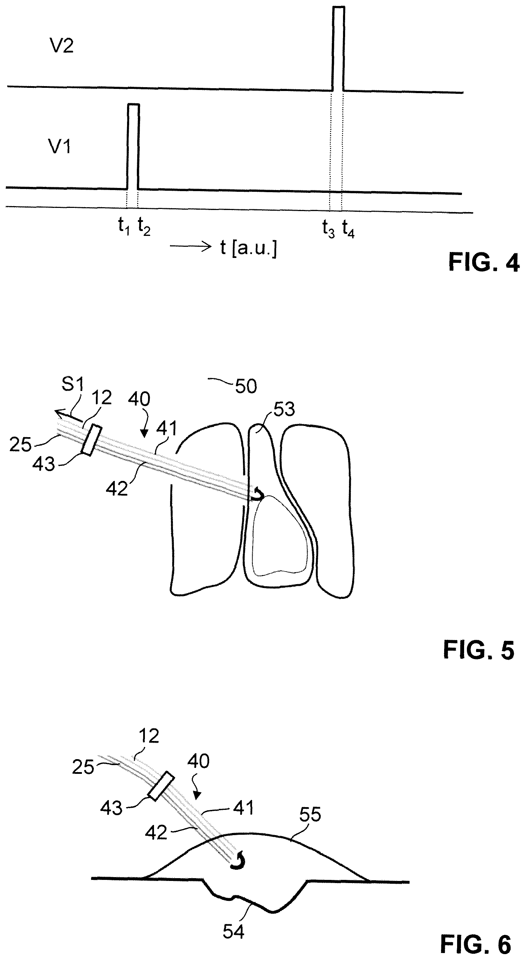

Um die Drainageleitung 12, 41 zu spülen, kann über die Hilfsleitung 25, 42 entkeimte Luft aus der Entkeimungszelle 30 zum vorrichtungsfernen Ende der Drainageleitung 12, 41 geleitet werden (Pfeil S2). Diese Luft gelangt in die Drainageleitung 12, 41, wird durch die Saugpumpe 18 angesaugt und spült dadurch die Drainageleitung 12, 41. Dadurch werden feste Bestandteile in der Drainageleitung 12, 41 mitgerissen und Okklusionen der Drainageleitung vermieden. Ein möglicher Ablauf eines solchen Spülvorgangs wird nachfolgend im Zusammenhang mit der Fig. 4 noch näher erläutert.

Die Fig. 2 illustriert die erwähnte Entkeimungszelle 30. Diese weist einen zylindrischen Seitenwandbereich 31 und einen darin dichtend verschiebbaren Kolben 32 auf. Dadurch ist in der Entkeimungszelle ein veränderliches Volumen gebildet. Durch eine Federanordnung 33 ist der Kolben 32 in Richtung vergrösserten Volumens vorbelastet. Die Federanordnung wirkt hier als Zugfeder. Es kann aber z.B. auch eine Druckfeder im Innern der Entkeimungszelle vorgesehen sein. Die Entkeimungszelle 30 weist einen Einlass 35 auf, an dem ein Einlassventil V1 angeordnet ist, sowie einen Auslass 36, an dem ein Auslassventil V2 angeordnet ist. In der Entkeimungszelle befindet sich als Behandlungseinrichtung eine Wärmequelle 34, die hier symbolisch durch eine Glühlampe angedeutet ist. Bei der Wärmequelle kann es sich um ein an sich bekanntes Heizelement beliebiger Art handeln, insbesondere um einen Dickschichtheizer, um eine Infrarot-Strahlungsquelle oder um ein beliebiges anderes Mittel, um das vom Seitenwandbereich 31 und Kolben 32 begrenzte Luftvolumen zu erwärmen. Das Luftvolumen wird vorzugsweise auf eine Temperatur von mindestens 70 Grad Celsius, bevorzugt mindestens 100 Grad Celsius, insbesondere bevorzugt mindestens 120 Grad Celsius erhitzt. Dadurch werden Keime im Luftvolumen abgetötet. Zur Überwachung kann in der Entkeimungszelle ein Temperatursensor vorgesehen sein.

Eine weitere Variante einer Entkeimungszelle 30' ist in der Fig. 3 dargestellt. Auch diese Entkeimungszelle 30' weist ein veränderliches Volumen auf. Dazu ist die Entkeimungszelle 30' mit einem faltenbalgartigen Wandbereich 31' versehen. Dadurch kann der Abstand einer Deckwand 32' der Entkeimungszelle 30' zum Einlass 35 und Auslass 36 verändert werden, um das in der Entkeimungszelle aufgenommene Luftvolumen zu verändern. Auch in dieser Variante kann optional eine Federanordnung vorgesehen sein, die an der Deckwand 32' angreift und danach trachtet, das Volumen der Entkeimungszelle zu maximieren, oder der faltenbalgartige Wandbereich 31' kann so ausgebildet sein, dass er eine derartige Federkraft erzeugt. Statt einer Wärmequelle 34 ist in der Entkeimungszelle 30' als Behandlungseinrichtung eine UV-Quelle 38 angeordnet, die in der Fig. 3 nur schematisch als UV-Leuchtdiode angedeutet ist. Statt einer UV-Leuchtdiode kann es sich bei der UV-Quelle auch um eine UV-Strahlung abgebende Gasentladungslampe oder eine beliebige andere Quelle von UV-Licht genügender Intensität handeln. Die UV-Quelle kann auch ausserhalb der eigentlichen Entkeimungszelle angeordnet sein, wenn die Entkeimungszelle ein UV-durchlässiges Fenster aufweist. Durch das erzeugte UV-Licht werden Keime in der Entkeimungszelle abgetötet.

Selbstverständlich kann eine UV-Lichtquelle auch in einer Entkeimungszelle mit beweglichem Kolben wie in der Fig. 2 eingesetzt werden, und eine Wärmequelle kann auch in einer Entkeimungszelle mit faltenbalgartigen Seitenwandbereich eingesetzt werden. Alternativ oder zusätzlich ist es z.B. auch denkbar einen Dampfgenerator vorzusehen, welcher heissen Wasserdampf in die Entkeimungszelle pumpt, einen Ozongenerator vorzusehen, der Ozon in die Entkeimungszelle einleitet, oder eine Elektronenquelle oder eine Gamma-Strahlungsquelle vorzusehen.

In der Fig. 4 ist illustriert, auf welche Weise die Entkeimungszelle der Figuren 2 oder 3 betrieben werden kann. Anfänglich herrscht in der Entkeimungszelle ein Unterdruck. Dadurch befindet sich die Entkeimungszelle in einem Zustand mit minimiertem Volumen (Figuren 2(a) und 3(a)). Nun wird zu einem Zeitpunkt t₁ das Ventil V1 geöffnet. Dadurch kann durch den Einlass 35 Luft vom Aussenraum in die Entkeimungszelle 30 einströmen. Aufgrund dieses Druckausgleichs nimmt die Entkeimungszelle einen Zustand ein, in dem ihr Volumen maximiert ist (Fig. 2(b) und Fig. 3(b)). Dabei wird die Entkeimungszelle durch die Federanordnung unterstützt. Das Ventil V1 wird nun zu einem Zeitpunkt t₂ wieder geschlossen. Nun wird die Behandlungseinrichtung in Form der Wärmequelle 34 oder der UV-Quelle 38 aktiviert, um Krankheitserreger, die möglicherweise mit der angesaugten Luft in die Entkeimungszelle gelangt sind, abzutöten. Nach einer genügend langen Einwirkzeit wird die Behandlungseinrichtung wieder deaktiviert und gegebenenfalls gewartet, bis sich die Luft in der Entkeimungszelle wieder abgekühlt hat. Zu einem Zeitpunkt t₃ wird das Auslassventil V2 geöffnet. Die Entkeimungszelle steht nun über die Auslassleitung 22, den Hilfsanschluss 24 und die Hilfsleitung 25, 42 in Verbindung mit der Drainageleitung 12, 41. Dadurch wird die Drainageleitung 12, 41 wie oben beschrieben gespült. Gleichzeitig entsteht so ein Unterdruck in der Entkeimungszelle, der dazu führt, dass die Entkeimungszelle in ihren Ausgangszustand mit minimiertem Volumen zurückkehrt (Fig. 2(a) und Fig. 3(a)). Das Auslassventil V2 wird zu einem Zeitpunkt t₄ wieder geschlossen, und ein neuer Entkeimungszyklus kann beginnen.

Optional kann die Entkeimungszelle mit einem Antrieb 37 ausgestattet sein, wie er in der Fig. 2 gestrichelt dargestellt ist. Der Antrieb 37 ermöglicht es, das Volumen der Entkeimungszelle aktiv zu verändern. Dazu kann z.B. der verschiebbare Kolben 32 mit einer Kolbenstange versehen sein, die als Zahnstange ausgebildet ist, wobei ein Antriebsmotor diese Zahnstange antreibt, um den Kolben 32 im Seitenwandbereich 31 aktiv zu verschieben. Statt auf den Kolben 32 kann eine Kolbenstange auch auf die Deckwand 32' in der Ausgestaltung der Fig. 3 einwirken. Der Antrieb 37 dient einerseits dazu, das Einsaugen von Luft aus der Umgebung durch die Einlassleitung 21 hindurch aktiv zu unterstützen. Andererseits kann der Antrieb dazu eingesetzt werden, die in der Entkeimungszelle aufgenommene Luft nach der Behandlung aktiv auszustossen. Auf diese Weise kann z.B. kurzzeitig ein Überdruck (z.B. ein Druckpuls) in der Hilfsleitung 25, 42 erzeugt werden, welcher dazu führt, dass sich die Druckdifferenz zwischen dem körpernahen Ende der Drainageleitung 12, 41 und dem Fluidsammelbehälter kurzzeitig erhöht. Dies unterstützt den Transport von Gewebeklumpen oder anderem Material in Richtung des Fluidsammelbehälters 11.

In den Figuren 5 und 6 sind weitere Anwendungsbeispiele für eine erfindungsgemässe Absaugvorrichtung illustriert. So ist in der Fig. 5 der doppellumige Katheter 40 in das Mediastinum eingeführt. In der Fig. 6 mündet der doppellumige Katheter 40 in einen Bereich oberhalb einer Wunde 54, die mit einer fluiddichten Abdeckung 55 abgedeckt ist. Insbesondere in dieser Ausführungsform kann der Katheter 40 auch vollständig entfallen, und der Drainageschlauch 12 sowie der Hilfsschlauch 25 können unmittelbar in den Bereich über der Wunde geführt sein. Eine Vielzahl anderer Ausgestaltungen am Ende der Drainageleitung und der Hilfsleitung sind denkbar. So ist es z.B. denkbar, die durch die Hilfsleitung 25, 42 zugeführte Luft grossflächig auf die Wunde zu leiten und zentral zur Drainageleitung 12, 41 hin abzusaugen.

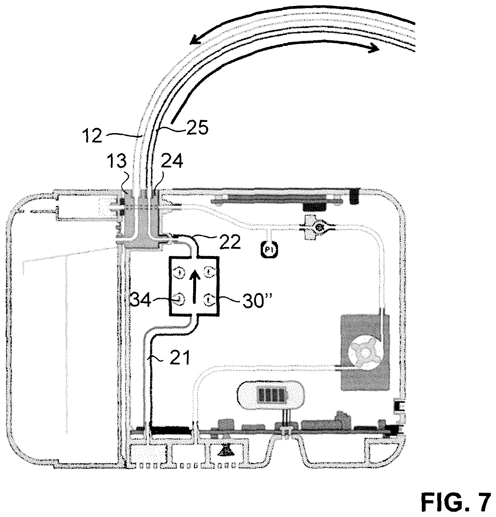

In der Fig. 7 ist eine weitere Ausführungsform einer Absaugvorrichtung mit Entkeimungszelle illustriert. Die Entkeimungszelle der Fig. 7 ist als reine Durchflusszelle 30" mit konstantem Volumen ausgebildet. Im vorliegenden Beispiel sind keinerlei Ventile vorgesehen. Es ist aber denkbar, zumindest am Einlass oder am Auslass der Entkeimungszelle ein Ventil vorzusehen, um zu steuern, wann der Hilfsleitung 25 entkeimte Luft zugeführt werden soll.

Die Entkeimungszelle kann in allen Ausführungsformen entweder fest in das Pumpaggregatsgehäuse integriert sein, oder sie kann nachrüstbar und/oder austauschbar sein. Dazu kann die Entkeimungszelle Verbindungselemente aufweisen, um die Entkeimungszelle mit dem Pumpaggregatsgehäuse 10 und/oder mit dem Fluidsammelbehälter 11 lösbar zu verbinden. Derartige Verbindungsstrukturen können z.B. Schnapprasten umfassen.

Selbstverständlich sind vielerlei Modifikationen der dargestellten Ausführungsbeispiele möglich, ohne den Bereich der vorliegenden Erfindung zu verlassen, und die Erfindung ist keineswegs auf die Ausführungsbeispiele beschränkt. Insbesondere kann der Drainageanschluss z.B. auch direkt am Fluidsammelbehälter ausgebildet sein. Die Absaugvorrichtung muss nicht notwendig eine tragbare, digitale Vorrichtung sein, sondern kann auch eine Vorrichtung herkömmlicher Art zum Anschluss an ein Spitalvakuumsystem sein. Statt eines Fluidsammelbehälters der hier dargestellten Art kann dementsprechend auch ein herkömmliches Fluidsammelsystem mit Wasserschloss vorgesehen sein (Ein-, Zwei- oder Dreiflaschensystem), wie dies aus dem Stand der Technik seit langem bekannt ist.

**BEZUGSZEICHENLISTE**

| | | | |
|---|---|---|---|
| 1 | Absaugvorrichtung | 35 | Einlass |
| 10 | Pumpaggregatsgehäuse | 36 | Auslass |
| 11 | F luidsammelbehälter | 37 | Antrieb |
| 12 | Drainageleitung | 38 | UV-Quelle |
| 13 | Drainageanschluss | 30' | Entkeimungszelle |
| 14 | Filter | 31' | faltenbalgartiger Wandbereich |
| 15 | Vakuumleitung | | |
| 16 | Druckaufnehmer | 32' | Deckwand |
| 17 | Einwegventil | 30" | Durchflusszelle |
| 18 | Saugpumpe | 40 | Katheter |
| 19 | Abluftleitung | 41 | Drainagelumen |
| 20 | Einlassfilter | 42 | Hilfslumen |
| 21 | Einlassleitung | 50 | Patient |
| 22 | Auslassleitung | 51 | Lungenflügel |
| 23 | Druckaufnehmer | 52 | Pleuraraum |
| 24 | Hilfsanschluss | 53 | Mediastinum |
| 25 | Hilfsleitung | 54 | Wunde |
| 27 | Batterie | 55 | Wundabdeckung |
| 28 | Steuerungseinrichtung | S1, S2 | Pfeil |
| 29 | Display | S3 | Doppelpfeil |
| 30 | Entkeimungszelle | V1 | Einlassventil |
| 31 | Seitenwandbereich | V2 | Auslassventil |
| 32 | Kolben | t | Zeit |
| 33 | Federanordnung | a.u. | willkürliche Einheiten |
| 34 | Wärmequelle | t₁-t₄ | Zeitpunkte |

## Patentansprüche

1. Absaugvorrichtung zum Absaugen eines Fluids aus einem menschlichen oder tierischen Körper (50) mittels Unterdruck, wobei die Absaugvorrichtung aufweist:
ein Pumpaggregatsgehäuse (10);
einen Drainageanschluss (13) für eine Drainageleitung (12) zum Abführen des Fluids aus dem Körper (50);
eine im Pumpaggregatsgehäuse (10) angeordnete Vakuumquelle (18) zur Erzeugung eines Unterdrucks am Drainageanschluss (13), um das Fluid durch die Drainageleitung (12) abzusaugen;
einen Fluidsammelbehälter (11) zum Sammeln des angesaugten Fluids; und
einen Hilfsanschluss (24) für eine Hilfsleitung (25),
**dadurch gekennzeichnet,**
**dass** die Absaugvorrichtung ausserdem eine Entkeimungseinrichtung mit einer im Pumpaggregatsgehäuse (10) angeordneten Entkeimungszelle (30) aufweist, wobei die Entkeimungszelle einen Einlass (35) aufweist, um ein Gas zur Entkeimungszelle (30) zuzuführen, wobei die Entkeimungseinrichtung (30) dazu ausgebildet ist, das zugeführte Gas derart zu behandeln, dass darin enthaltene Pathogene verändert und dadurch unschädlich gemacht werden, und wobei die Entkeimungszelle einen Auslass (36) aufweist, um das behandelte Gas aus der Entkeimungszelle (30) zum Hilfsanschluss (24) abzuführen.

2. Absaugvorrichtung nach Anspruch 1, **dadurch gekennzeichnet, dass** der Einlass (35) mit einem Aussenraum verbunden ist, um Umgebungsluft zur Entkeimungszelle (30) zuzuführen.

3. Absaugvorrichtung nach Anspruch 1 oder 2, **dadurch gekennzeichnet, dass** die Entkeimungseinrichtung eine Behandlungseinrichtung aufweist, um das zugeführte Gas mittels UV-Strahlen, durch Temperaturerhöhung und/oder durch Einwirkung von Wasserdampf zu behandeln, wobei die Behandlungseinrichtung bevorzugt mindestens eine UV-Lichtquelle (38) und/oder mindestens eine Wärmequelle (34) umfasst.

4. Absaugvorrichtung nach einem der vorhergehenden Ansprüche, **dadurch gekennzeichnet, dass** die Entkeimungseinrichtung am Einlass der Entkeimungszelle (30) ein Einlassventil (V1) und am Auslass der Entkeimungszelle (30) ein Auslassventil (V2) aufweist.

5. Absaugvorrichtung nach Anspruch 4, **dadurch gekennzeichnet, dass** die Absaugvorrichtung eine Steuerungseinrichtung (28) aufweist, welche dazu ausgebildet ist, die folgenden Schritte durchzuführen:
(a) Schliessen des Auslassventils (V2);
(b) Öffnen des Einlassventils (V1), um das Gas zur Entkeimungszelle zuzuführen;
(c) Schliessen des Einlassventils (V1);
(d) Behandeln des zugeführten Gases in der Entkeimungszelle (30) derart, dass darin enthaltene Pathogene verändert und dadurch unschädlich gemacht werden; und
(e) Öffnen des Auslassventils (V2), um das behandelte Gas aus der Entkeimungszelle (30) abzuführen,
wobei Schritt (b) vor, während oder nach Schritt (a) durchgeführt wird, wobei Schritt (c) nach Schritt (b) durchgeführt wird, und wobei Schritt (d) zumindest zwischen Schritt (c) und Schritt (e) durchgeführt wird.

6. Absaugvorrichtung nach einem der vorhergehenden Ansprüche, **dadurch gekennzeichnet, dass** die Entkeimungszelle (30) ein veränderliches Volumen aufweist.

7. Absaugvorrichtung nach Anspruch 6, **dadurch gekennzeichnet, dass** die Entkeimungszelle einen zylindrischen Wandbereich (31), der vorteilhaft elastisch oder faltenbalgartig ist, und einen darin verschiebbaren Kolben (32) aufweist, um das Volumen der Entkeimungszelle zu verändern.

8. Absaugvorrichtung nach einem der vorhergehenden Ansprüche, **dadurch gekennzeichnet, dass** die Entkeimungszelle als Durchflusszelle (30") mit konstantem Volumen ausgebildet ist.

9. Absaugvorrichtung nach einem der vorhergehenden Ansprüche, **dadurch gekennzeichnet,**
**dass** die Absaugvorrichtung eine Drainageleitung (12) aufweist, die mit dem Drainageanschluss (13) verbunden ist,
**dass** die Absaugvorrichtung eine Hilfsleitung (25) aufweist, die mit dem Hilfsanschluss (24) verbunden ist, und
**dass** ein vorrichtungsfernes Ende der Hilfsleitung (25) mit einem vorrichtungsfernen Ende der Drainageleitung (12) in fluidkommunizierender Verbindung steht.

10. Absaugvorrichtung nach einem der vorhergehenden Ansprüche, **dadurch gekennzeichnet, dass** die Entkeimungszelle (30) nachrüstbar und/oder austauschbar ist.

## Claims

1. A suction apparatus for aspirating a fluid from a human or animal body (50) by means of negative pressure, the suction apparatus comprising:
a pump assembly housing (10);
a drainage connection (13) for a drainage line (12) for removing the fluid from the body (50);
a vacuum source (18) arranged in the pump assembly housing (10) for generating a negative pressure at the drainage connection (13) in order to suck up the fluid through the drainage line (12);
a fluid collection container (11) for collecting the sucked-up fluid; and
an auxiliary connection (24) for an auxiliary line (25),
**characterized in that**
the suction apparatus further comprises a sterilizing device with a sterilizing cell (30) arranged in the pump assembly housing (10), wherein the sterilizing cell has an inlet (35) in order to supply a gas to the sterilizing cell (30), wherein the sterilizing device (30) is configured to treat the supplied gas in such a manner that pathogens contained in said gas are modified and thereby rendered harmless, and wherein the sterilizing cell has an outlet (36) in order to conduct the treated gas out of the sterilizing cell (30) to the auxiliary connection (24).

2. The suction apparatus according to Claim 1, **characterized in that** the inlet (35) is connected to an environmental space in order to supply ambient air to the sterilizing cell (30).

3. The suction apparatus according to Claim 1 or 2, **characterized in that** the sterilizing device comprises a treatment device in order to treat the supplied gas by means of UV rays, by increasing the temperature and/or by the action of steam, wherein the treatment device preferably comprises at least one UV light source (38) and/or at least one heat source (34).

4. The suction apparatus according to any one of the preceding claims, **characterized in that** the sterilizing device comprises an inlet valve (V1) at the inlet of the sterilizing cell (30) and an outlet valve (V2) at the outlet of the sterilizing cell (30).

5. The suction apparatus according to Claim 4, **characterized in that** the suction apparatus comprises a control device (28) which is configured to carry out the following steps:
(a) closing the outlet valve (V2);
(b) opening the inlet valve (V1) in order to supply the gas to the sterilizing cell;
(c) closing the inlet valve (V1);
(d) treating the supplied gas in the sterilizing cell (30) in such a manner that pathogens contained in said gas are modified and thereby rendered harmless; and
(e) opening the outlet valve (V2) in order to conduct the treated gas out of the sterilizing cell (30),
wherein step (b) is carried out before, during or after step (a), wherein step (c) is carried out after step (b), and wherein step (d) is carried out at least between step (c) and step (e).

6. The suction apparatus according to any one of the preceding claims, **characterized in that** the sterilizing cell (30) has a variable volume.

7. The suction apparatus according to Claim 6, **characterized in that** the sterilizing cell has a cylindrical wall region (31), which advantageously is elastic or expansion-bellows-like, and a plunger (32) which is displaceable therein in order to change the volume of the sterilizing cell.

8. The suction apparatus according to any one of the preceding claims, **characterized in that** the sterilizing cell is designed as a continuous flow cell (30") having a constant volume.

9. The suction apparatus according to any one of the preceding claims, **characterized**
**in that** the suction apparatus comprises a drainage line (12) which is connected to the drainage connection (13),
**in that** the suction apparatus comprises an auxiliary line (25) which is connected to the auxiliary connection (24), and
**in that** an apparatus-remote end of the auxiliary line (25) is in fluid-communicating connection with an apparatus-remote end of the drainage line (12).

10. The suction apparatus according to any one of the preceding claims, **characterized in that** the sterilizing cell (30) is retrofittable and/or interchangeable.

## Revendications

1. Un dispositif d'aspiration servant à aspirer un fluide hors d'un organisme humain ou animal (50) au moyen de pression négative, où le dispositif d'aspiration présente :
un boîtier de groupe de pompage (10) ;
un raccord de drainage (13) pour une conduite de drainage (12) servant à évacuer le fluide hors de l'organisme (50) ;
une source de vide (18) disposée dans le boîtier de groupe de pompage (10) servant à générer une pression négative au niveau du raccord de drainage (13) pour aspirer le fluide à travers la conduite de drainage (12) ;
un récipient de collecte de fluide (11) servant à collecter le fluide aspiré ; et
un raccord auxiliaire (24) pour une conduite auxiliaire (25),
**caractérisé en ce que**,
le dispositif d'aspiration présente en outre une installation de désinfection ayant une cellule de désinfection (30) disposée dans boîtier de groupe de pompage (10), où la cellule de désinfection (30) présente une entrée (35) pour acheminer un gaz vers la cellule de désinfection (30), où l'installation de désinfection (30) est configurée de sorte à traiter le gaz acheminé de façon à modifier les pathogènes qu'il contient et les rendre ainsi inoffensifs, et où la cellule de désinfection présente une sortie (36) pour évacuer le gaz traité hors de la cellule de désinfection (30) vers le raccord auxiliaire (24).

2. Le dispositif d'aspiration selon la revendication 1, **caractérisé en ce que** l'entrée (35) est connectée à un espace extérieur pour acheminer de l'air environnant vers la cellule de désinfection (30).

3. Le dispositif d'aspiration selon la revendication 1 ou 2, **caractérisé en ce que** l'installation de désinfection présente une installation de traitement, pour traiter le gaz acheminé au moyen de rayons UV, par augmentation de température et/ou par action de vapeur d'eau, où l'installation de traitement comprend préférablement au moins une source de lumière UV (38) et/ou au moins une source de chaleur (34).

4. Le dispositif d'aspiration selon une des revendications précédentes, **caractérisé en ce que** l'installation de désinfection présente une soupape d'entrée (V1) au niveau de l'entrée de la cellule de désinfection (30) et une soupape de sortie (V2) au niveau de la sortie de la cellule de désinfection (30).

5. Le dispositif d'aspiration selon la revendication 4, **caractérisé en ce que** le dispositif d'aspiration présente une installation de commande (28), laquelle est configurée pour effectuer les étapes suivantes :
(a) fermeture de la soupape de sortie (V2) ;
(b) ouverture de la soupape d'entrée (V1) pour acheminer le gaz vers la cellule de désinfection ;
(c) fermeture de la soupape d'entrée (V1) ;
(d) traitement du gaz acheminé dans la cellule de désinfection (30) de sorte à modifier les pathogènes qu'ils contient et les rendre ainsi inoffensifs ; et
(e) ouverture de la soupape de sortie (V2), pour évacuer le gaz traité hors de la cellule de désinfection (30),
où l'étape (b) est effectuée avant, lors de ou après l'étape (a), ou l'étape (c) est effectuée après l'étape (b), et où l'étape (d) est au moins effectuée entre l'étape (c) et l'étape (e).

6. Le dispositif d'aspiration selon une des revendications précédentes, **caractérisé en ce que** la cellule de désinfection (30) présente un volume variable.

7. Le dispositif d'aspiration selon la revendication 6, **caractérisé en ce que** la cellule de désinfection présente une zone de paroi cylindrique (31), qui est préférablement élastique ou du type soufflet, ainsi qu'un piston (33) pouvant être coulissé à l'intérieur de celle-ci, pour varier le volume de la cellule de désinfection.

8. Le dispositif d'aspiration selon une des revendications précédentes, **caractérisé en ce que** la cellule de désinfection est formée en tant que cellule à flux traversant (30") ayant un volume constant.

9. Le dispositif d'aspiration selon une des revendications précédentes, **caractérisé en ce que** le dispositif d'aspiration présente une conduite de drainage (12), qui est connectée au raccord de drainage (13), que le dispositif d'aspiration présente une conduite auxiliaire (25), qui est connectée au raccord auxiliaire (24), et qu'une extrémité éloignée par rapport au dispositif de la conduite auxiliaire (25) est en connexion fluidiquement communicante avec une extrémité éloignée par rapport au dispositif de la conduite de drainage (12).

10. Le dispositif d'aspiration selon une des revendications précédentes, **caractérisé en ce que** la cellule de désinfection (30) est échangeable et/ou modifiable.
